# EUROPEAN PATENT SPECIFICATION

(11) **EP 4 038 084 B1**
(45) Date of publication and mention of the grant of the patent: **01.11.2023**
(21) Application number: 20800296.4
(22) Date of filing: 30.09.2020
(51) Int. Cl.: C07K 5/107, C07K 5/117, A61P 31/04

(54) **LIPOPEPTIDES, PHARMACEUTICAL COMPOSITION, COSMETIC COMPOSITION, AND LIPOPEPTIDES FOR USE AS A DRUG**
LIPOPEPTIDE, PHARMAZEUTISCHE ZUSAMMENSETZUNG, KOSMETISCHE ZUSAMMENSETZUNG UND LIPOPEPTIDE ZUR VERWENDUNG ALS ARZNEIMITTEL
LIPOPEPTIDES, COMPOSITION PHARMACEUTIQUE, COMPOSITION COSMÉTIQUE ET LIPOPEPTIDES DESTINÉS À ÊTRE UTILISÉS EN TANT QUE MÉDICAMENT

(30) Priority: 01.10.2019 PL 43133619
(43) Date of publication of application: 10.08.2022
(73) Proprietor: Uniwersytet Warszawski, 00-927 Warszawa (PL)
(72) Inventor: SEK, Slawomir, 02-797 Warszawa (PL); TYMECKA, Dagmara, 02-384 Warszawa (PL); JUHANIEWICZ-DEBINSKA, Joanna, 00-710 Warszawa (PL); BARTOSIK, Dariusz, 02-495 Warszawa (PL); LASEK, Robert, 02-094 Warszawa (PL)
(74) Representative: JD&P Patent Attorneys Joanna Dargiewicz & Partners
(86) International application number: PCT/IB2020/059149
(87) International publication number: WO 2021/064593

(56) References cited:
- GAYAN HERUKA DE ZOYSA ET AL: "Feasibility Study Exploring the Potential of Novel Battacin Lipopeptides as Antimicrobial Coatings", ACS APPLIED MATERIALS & INTERFACES, vol. 9, no. 2, 18 January 2017 (2017-01-18), pages 1373-1383, XP055758683, US ISSN: 1944-8244, DOI: 10.1021/acsami.6b15859
- GAYAN HERUKA DE ZOYSA ET AL: "Unexplored antifungal activity of linear battacin lipopeptides against planktonic and mature biofilms of C. albicans", EUROPEAN JOURNAL OF MEDICINAL CHEMISTRY, vol. 146, 1 February 2018 (2018-02-01), pages 344-353, XP55758662, NL ISSN: 0223-5234, DOI: 10.1016/j.ejmech.2018.01.023
- JOANNA JUHANIEWICZ-DEBINSKA ET AL: "Lipopeptide-induced changes in permeability of solid supported bilayers composed of bacterial membrane lipids", JOURNAL OF ELECTROANALYTICAL CHEMISTRY, vol. 812, 1 March 2018 (2018-03-01), pages 227-234, XP55758668, AMSTERDAM, NL ISSN: 1572-6657, DOI: 10.1016/j.jelechem.2017.12.065 cited in the application

## Description

The present invention relates to novel linear ultrashort lipopeptides, pharmaceutical and cosmetic compositions comprising lipopeptides, and lipopeptides for use as drugs in the treatment of bacterial infections.

The increasing number of cases of bacterial drug resistance constitutes nowadays a significant clinical problem, hence the growing interest in compounds that could be an effective alternative, while showing different mechanisms of action than that of the traditional antibiotics. A group of compounds that could potentially meet these requirements are lipopeptides.

Unlike traditional antibiotics which act specifically, for example by interfering with the activity of enzymes involved in the cell wall synthesis or by damaging DNA, the lipopeptides show the ability to penetrate and irreversibly destroy the bacterial cell membrane structure (see, for example, Strauss, S.K.; Hancock, R.E.W. "Mode of action of the new antibiotic for Gram-positive pathogens daptomycin: comparison with cationic antimicrobial peptides and lipopeptides" Biochim. Biophys. Acta (BBA) - Biomembranes 1758: 1215-1223 (2006)). Since their action is less specific than that of the traditional drugs, there is a lower probability of resistance to these types of compounds.

To this class of compounds belongs daptomycin, which is a natural lipopeptide and exhibits a potent bactericidal activity *in vitro* and *in vivo* against clinically important Gram-positive bacteria that can cause serious and life-threatening diseases, as described for example in the publication of Tally *et al*. (Tally, F.P. et al., "Daptomycin: a novel agent for Gram-positive infections," Exp. Opin. Invest. Drugs 8: 1223-1238 (1999)).

Classes of lipopeptide compounds that have shown significant potential as useful antibiotics include the lipopeptides described, for example, in U.S. Patent No. 6,911,525 and U.S. 2015/0080292. De Zoysa et al., in "Feasibility Study Exploring the Potential of Novel Battacin Lipopeptides as Antimicrobial Coatings" ACS Appl. Mater. Interfaces 9 (2017) 1373-1383, describe antibiotic surfaces modified with Battacin, a cyclic lipopeptide antibiotic which is active against multidrug-resistant gram-negative bacteria.

Juhaniewicz-D 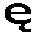 bińska *et al*., in "Lipopeptide-induced changes in permeability of solid supported bilayers composed of bacterial membrane lipids" Journal of Electroanalytical Chemistry 812 (2018) 227-234, described an ultrashort lipopeptide with the formula C₁₅H₃₁CO-DPhe-Dab-Dab-Leu-NH₂ and its effect on a simplified model mimicking the membrane system of *E*. *coli*, a Gram-negative bacteria. Although the studies showed C₁₅H₃₁CO-DPhe-Dab-Dab-Leu-NH₂ lipopeptide activity in that model, it should be remembered, though, that that model did not include the majority of natural membrane components and the analyzes performed on models did not reveal whether a given compound could exhibit antibacterial properties.

Most of the lipopeptides described in the literature are of natural origin, which entails the costs of isolation from biological material. Moreover, most of the mentioned lipopeptides have a cyclic, relatively complex structure, which in turn makes the possible synthesis and further modifications complicated.

The object of the invention is to provide ultrashort linear lipopeptides that show antibacterial activity against Gram-positive and Gram-negative bacteria. For purposes of this specification, when referring to amino acid residues the standard nomenclature of three-letter or one-letter abbreviations is used. In the case of one-letter designations of amino acid residues, L-amino acid residues are designated with capital letters, while D-amino acid residues are designated with lower case letters. D-tryptophan amino acid residue is designated with the symbol "w", D-phenylalanine - with the symbol "f", L-leucine - with the symbol "L", L-2,4-diaminobutyric acid - with the symbol "X", and L-alanine - with the symbol "A". In case of three-letter designation, the amino acid residue of D-tryptophan is designated as "D-Trp", D-phenylalanine as "D-Phe", L-leucine as "Leu", L-2,4-diaminobutyric acid as "Dab", while L-alanine as "Ala". When referring to fatty acid residues, symbols designating the number of carbon atoms in the molecule are used. For example, the symbols C12, C14, and C16 represent dodecanoic acid, tetradecanoic acid, and hexadecanoic acid residues, respectively.

The lipopeptides of the invention can be described by the general Formula 1:

**CH₃(CH₂)ₙC(O)-Xaa1-Dab-Dab-Xaa2-NH₂** (Formula 1)

wherein:
CH₃(CH₂)ₙC(O)- is a saturated fatty acid residue, wherein n=10-14;
Xaa1 is D-tryptophan or D-phenylalanine amino acid residue;
Xaa2 is L-leucine or L-alanine amino acid residue;
Dab is L-2,4-diaminobutyric acid amino acid residue;
excluding CH₃(CH₂)₁₄C(O)-D-Phe-Dab-Dab-Leu-NH₂ lipopeptide,
wherein the lipopeptides may be in a free form or in form of any pharmaceutically or cosmetically acceptable salt.

Preferably, n=10-13, more preferably n=10 or n=12. The Xaa1 amino acid residue may be a D-phenylalanine residue, while the Xaa2 amino acid residue may be an L-leucine residue.

The pharmaceutical composition according to the invention comprises lipopeptides of the general Formula 1. Preferably, the composition comprises 0.1 to 99% wt. of lipopeptides of general Formula 1. The pharmaceutical composition may comprise pharmaceutically acceptable carriers and/or diluting agents and/or excipients and/or vehicles. For example, but not limited to, the compositions may comprise corn starch and/or gelatin and/or lactose and/or sucrose and/or microcrystalline cellulose and/or kaolin and/or mannitol and/or dicalcium phosphate and/or sodium chloride and/or alginic acid. Preferably, the pharmaceutical composition is in a form suitable for oral and/or intravenous and/or intramuscular and/or subcutaneous and/or parenteral administration, e.g., in form of tablets and/or capsules and/or elixir and/or suspension and/or syrup and/or gel and/or cream and/or ointment.

The cosmetic composition according to the invention comprises lipopeptides of general Formula 1 in any cosmetically acceptable dosage form. The cosmetic composition may comprise 0.1 to 99% wt. of lipopeptides of general Formula 1. The cosmetic composition optionally comprises a cosmetic carrier. Preferably, the cosmetic composition is in the form of gel or cream or suspension.

The invention also includes lipopeptides of general Formula 1 for use as a drug. Preferably, the lipopeptides of general Formula 1 are intended for the treatment of bacterial infections, preferably caused by Gram-positive bacteria, especially of the genus Staphylococcus.

The ultrashort lipopeptides of the invention may be obtained by any synthetic method known in the prior art. For example, said synthetic method may include liquid-phase synthesis or solid-phase synthesis.

The ultrashort lipopeptides according to the invention have an amphiphilic structure, and due to the presence of Dab residues, at physiological pH (about 7.4) they have an excessive positive charge, which facilitates their interaction with the bacterial cell membrane.

Due to their activity of inhibiting the growth of Gram-positive bacteria such as *Enterococcus faecalis, Staphylococcus aureus, Staphylococcus epidermidis,* and Gram-negative bacteria such as *Klebsiella pneumoniae, Pseudomonas aeruginosa, Yersinia enterocolitica,* the ultrashort lipopeptides according to the invention can be used in the treatment of bacterial infections. The ultrashort lipopeptides according to the invention show particular activity against bacteria of the genus *Staphylococcus*.

The advantage of the ultrashort lipopeptides according to the invention is their mechanism of action, based on direct interaction with the bacterial cell membrane, which in turn reduces the risk of drug resistance development. In addition, the described lipopeptides according to the invention are simple and easy-to-synthesize structures, which significantly reduces their production cost. Their adequate solubility in an aqueous medium is also ensured, which significantly broadens the range of possibilities regarding the dosage form of a possible pharmaceutical or cosmetic composition.

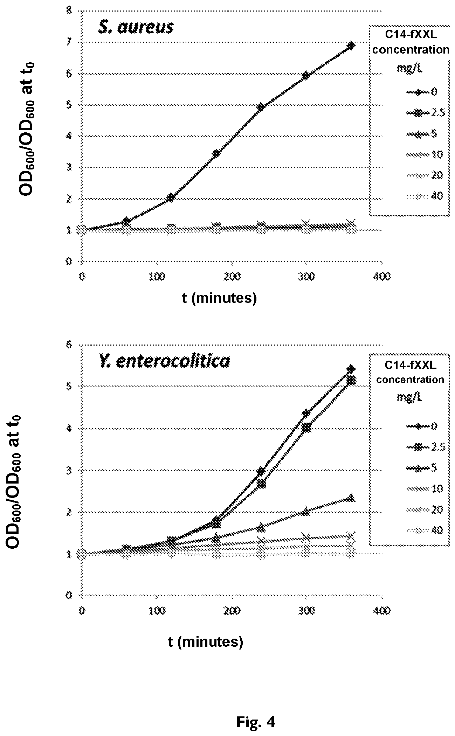

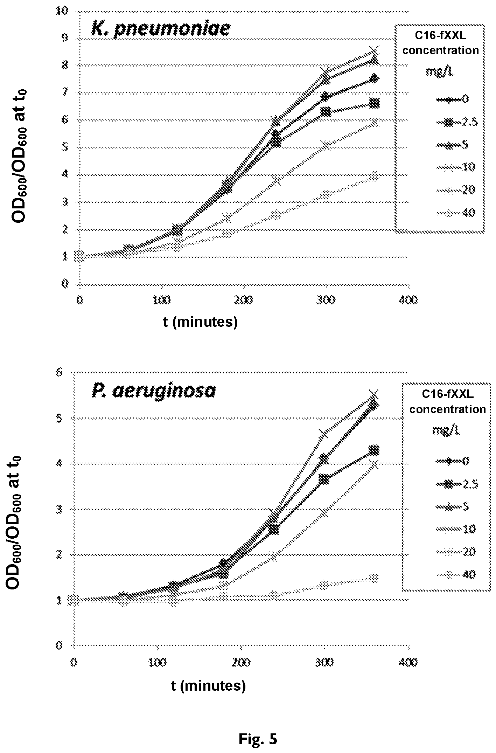

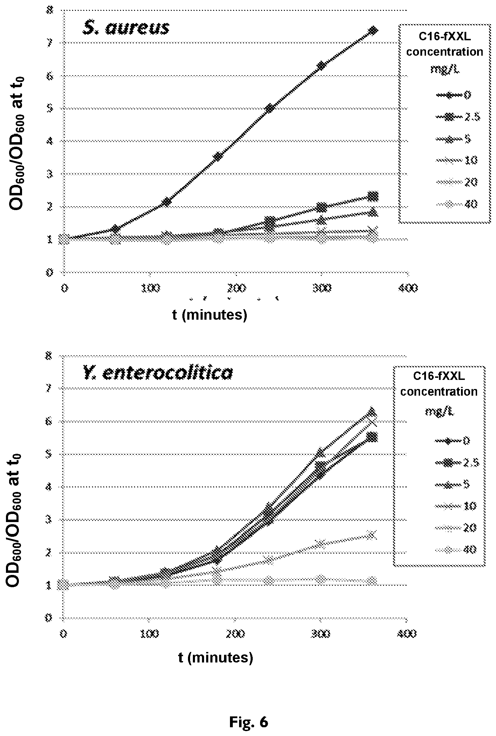

The subject-matter of the invention is illustrated in the embodiments in the drawing, where **Fig. 1** shows the effect of C12-fXXL lipopeptide on the growth dynamics of *Klebsiella pneumoniae* and *Pseudomonas aeruginosa* strains, expressed as the relative culture optical density against time; **Fig. 2** shows the effect of the C12-fXXL lipopeptide on the growth dynamics of *Staphylococcus aureus* and *Yersinia enterocolitica* strains, expressed as the relative culture optical density against time; **Fig. 3** shows the effect of C14-fXXL lipopeptide on the growth dynamics of *Klebsiella pneumoniae* and *Pseudomonas aeruginosa* strains, expressed as relative culture optical density against time; **Fig. 4** shows the effect of C14-fXXL lipopeptide on the growth dynamics of *Staphylococcus aureus* and *Yersinia enterocolitica* strains, expressed as relative culture optical density against time; **Fig. 5** shows the effect of C16-fXXL lipopeptide on the growth dynamics of *Klebsiella pneumoniae* and *Pseudomonas aeruginosa* strains, expressed as relative culture optical density against time; **Fig. 6** shows the effect of the C16-fXXL lipopeptide on the growth dynamics of *Staphylococcus aureus* and *Yersinia enterocolitica* strains, expressed as the relative culture optical density against time; **Fig. 7** shows a graph of hemolysis index (%) versus lipopeptide concentration (in µg/mL) using sheep red blood cells; **Fig. 8** shows a graph of the hemolysis index (%) versus the lipopeptide concentration (in µg/mL) using horse red blood cells.

To illustrate the invention, examples are included below, which, however, should not be interpreted as limiting the scope of the invention in any way.

### EXAMPLE 1

### Bacterial strains and culture media

All bacterial strains were obtained from the Polish Collection of Microorganisms (PCM) or the American Type Culture Collection (ATCC). Gram-positive bacteria strains: *Staphylococcus aureus* ATCC 29213, *Staphylococcus epidermidis* ATCC 12228, and *Enterococcus faecalis* ATCC 14506. Gram-negative bacteria strains: *Escherichia coli* ST2-8624 O157:H7, *Pseudomonas aeruginosa* PAO1 PCM 499, *Klebsiella pneumoniae* PCM 1, *Salmonella sv. Typhimurium* TT622, and *Yersinia enterocolitica* PCM 2081. All strains were cultured in LB (lysogenic broth) medium.

### Structure of the test lipopeptides according to the invention

The biological activity was tested for two lipopeptide compounds represented by Formula 1. More specifically, they were the following lipopeptides according to the invention:
(1) C12-fXXL: CH₃(CH₂)₁₀C(O)-D-Phe-Dab-Dab-Leu-NH₂
(2) C14-fXXL: CH₃(CH₂)₁₂C(O)-D-Phe-Dab-Dab-Leu-NH₂

The biological activity of compounds (1) and (2) was compared with the biological activity of the C16-fXXL: CH₃(CH₂)₁₄C(O)-D-Phe-Dab-Dab-Leu-NH₂ lipopeptide.

### Determination of the minimal inhibitory concentration (MIC)

10 mL of LB medium was inoculated with material from a single colony and cultured at 30°C overnight with shaking. Thereafter, the optical density of the overnight cultures (at 600 nm) was adjusted to 0.05 by dilution in a fresh portion of LB medium. The test compounds were dissolved in water. Serial dilutions of compounds were prepared in LB medium ranging from 5 to 50 µg/mL (final concentrations). The experiment was performed by adding 100 µL of each of the prepared dilutions to 100 µL of the diluted overnight bacterial culture in the wells of a 96-well titration plate. The MIC was determined as the lowest concentration of test compound needed to inhibit bacterial growth when assessed after 24 h incubation at 30°C with shaking (final optical density at 600 nm not greater than 0.05). Optical density measurements were performed using TECAN Sunrise plate reader. Data were obtained from three independent experiments. The obtained results are summarized in Table 2.

**Table 2**

| **Bacterial strain** | **MIC [µg/mL**= **mg/L]** | | |
|---|---|---|---|
| | **C12-fXXL** | **C14-fXXL** | **C16-fXXL** |
| *Enterococcus faecalis* ATCC 14506 | 30 | 30 | n.d. |
| *Escherichia coli* ST2-8624 O157:H7 | 50 | n.d. | n.d. |
| *Klebsiella pneumoniae* PCM 1 | 20 | 20 | n.d. |
| *Pseudomonas aeruginosa* PAO1 PCM 499 | 20 | 20 | n.d. |
| *Salmonella* sv. *Typhimurium* TT622 | n.d. | n.d. | n.d. |
| *Staphylococcus aureus* ATCC 29213 | 5 | 5 | 5 |
| *Staphylococcus epidermidis* ATCC 12228 | 5 | 5 | 5 |
| *Yersinia enterocolitica* PCM 2081 | 20 | 10 | 30 |

| | | | |
|---|---|---|---|
| MIC = minimum inhibitory concentration n.d. = not determined, i.e., the MIC value was greater than 50 µg/mL | | | |

The results of the measurements summarized in Table 2 indicate that all of the test lipopeptides exhibit variable activity against the test bacterial strains. C12-fXXL and C14-fXXL lipopeptides show activity against both Gram-positive and Gram-negative bacterial strains. C16-fXXL compound, not according to the invention and listed in the table for comparative purposes only, shows activity against only three out of eight analyzed bacterial strains. C16-fXXL compound shows no activity against *Escherichia coli* ST2-8624 O157:H7 strain, although its interaction with a model bilayer mimicking the *E*. *coli* membrane system is known from the literature (Juhaniewicz-D 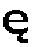 bińska *et al.*, "Lipopeptide-induced changes in the permeability of solid supported bilayers composed of bacterial membrane lipids." Journal of Electroanalytical Chemistry 812 (2018) 227-234).

However, one can talk here about the selectivity against Gram-positive strains of *Staphylococcus aureus* ATCC 29213, *Staphylococcus epidermidis* ATCC 12228, in case of which the highest activity of the test lipopeptides was observed, i.e., the lowest values of the minimum inhibitory concentration (MIC). At the same time, the activity of the test lipopeptides against *Escherichia coli* ST2-8624 O157:H7 and *Salmonella sv. Typhimurium* TT622 strains is minimal.

### Determination of the lipopeptide effect on the bacterial growth dynamics

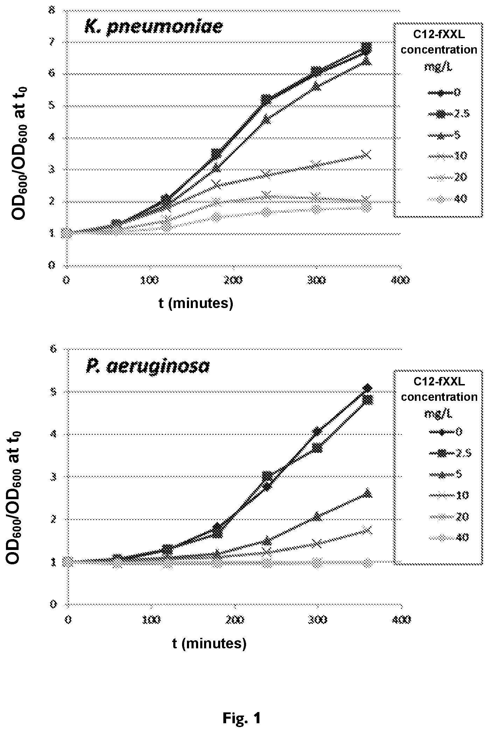

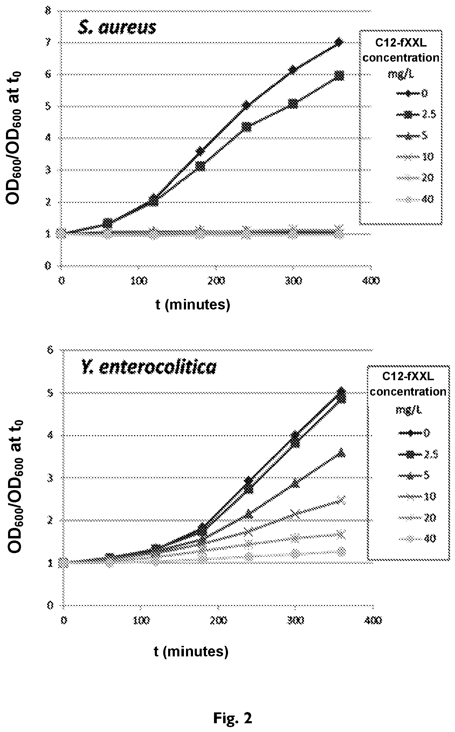

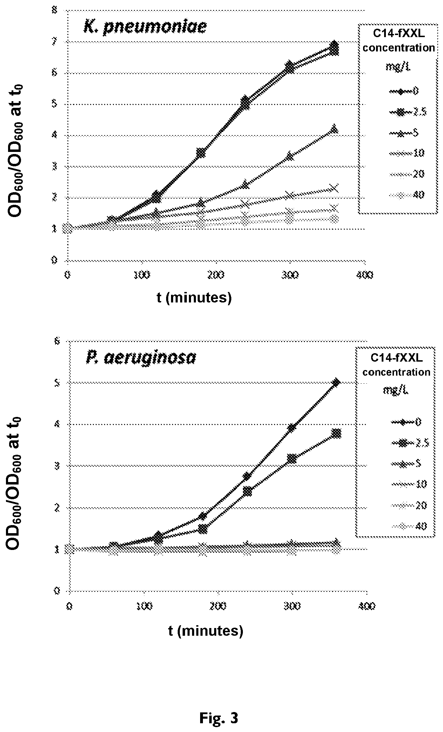

The effect of different concentrations of the test compounds on the bacterial growth was tested on 3 Gram-negative bacterial strains: *Klebsiella pneumoniae* PCM 1, *Pseudomonas aeruginosa* PAO1 PCM 499, and *Yersinia enterocolitica* PCM 2081 and one Gram-positive strain: *Staphylococcus aureus* ATCC 29213. The experiment was carried out in a similar way to the MIC determination, but in this case the diluted overnight cultures were pre-incubated for 2 h before adding diluted lipopeptide preparations. The bacterial growth was then monitored by measuring the optical density of the culture at 600 nm every 60 min for 6 h. Data were obtained from three independent experiments. The measurement results are shown as graphs in Figs. 1-3.

The obtained results show that the test lipopeptides significantly slow down the growth dynamics of the four test bacterial strains, while in the case of Gram-negative bacterial strains, C12-fXXL and C14-fXXL lipopeptides slowed down the bacterial growth dynamics at much lower concentrations than that of the C16-fXXL lipopeptide. However, the strongest effect of bacterial growth slowing down was observed for the Gram-positive bacteria strain: *Staphylococcus aureus* ATCC 29213, where lipopeptide concentrations of 2.5 to 5.0 mg/L caused almost complete inhibition of bacterial growth. An equally strong effect of slowing down the Gram-negative bacterial growth dynamics was also observed for the C14-fXXL lipopeptide against the *Pseudomonas aeruginosa* PAO1 PCM 499 strain.

### EXAMPLE 2

### Examination of hemolytic activity of lipopeptides

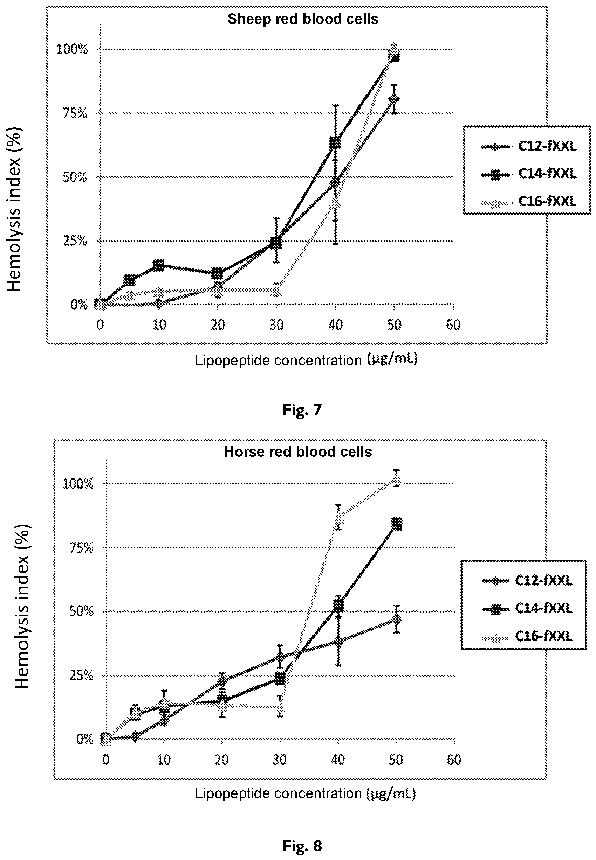

Defibrinated sheep or horse blood was washed three times in PBS buffer (137 mM NaCl, 2.7 mM KCl, 8 mM Na₂HPO₄, 2 mM KH₂PO₄, pH 7.4) and diluted to obtain 2% (v/v) preparation of red blood cells (RBC). Serial dilutions of compounds were prepared in PBS buffer medium ranging from 5 to 50 µg/mL (final concentrations). The experiment was performed by adding 190 µL of 2% RBC preparation to 10 µL of each of the prepared lipopeptide dilutions in the wells of a 96-well titer plate. As a positive control, Triton X-100 (2% final concentration) was used, instead of test compound preparations, and PBS buffer was used as a negative control. The samples were then incubated for 30 min at 30°C with shaking. After this time, the plate was centrifuged (1200 rpm) and 150 µL of the supernatant was transferred per well to another titration plate and the absorbance at 415 nm was measured using TECAN Sunrise plate reader. Based on the obtained results, the concentration of lipopeptides was determined at which up to 50% of hemolysis occurs in comparison with the positive control (HC₅₀). Data were obtained from three independent experiments. The measurement results are shown in Table 3 and in the form of graphs in Figs. 7 and 8.

**Table 3**

| **Lipopeptide** | **Sheep red blood cells** | **Horse red blood cells** |
|---|---|---|
| C12-fXXL | 30 µg/mL < HC₅₀< 40 µg/mL | 30 µg/mL < HC₅₀< 40 µg/mL |
| C14-fXXL | 30 µg/mL < HC₅₀< 40 µg/mL | 30 µg/mL < HC₅₀< 40 µg/mL |
| C16-fXXL | 30 µg/mL < HC₅₀< 40 µg/mL | 30 µg/mL < HC₅₀< 40 µg/mL |

The obtained results indicate that the test lipopeptides show moderate hemolytic activity at practically the same level. This applies to the results obtained with red blood cells from both sheep and horse.

## Claims

1. Lipopeptides of general Formula 1:
**CH₃(CH₂)ₙC(O)-Xaa1-Dab-Dab-Xaa2-NH₂** (Formula 1)
wherein:
CH₃(CH₂)ₙC(O)- is a saturated fatty acid residue, wherein n=10-14;
Xaa1 is D-tryptophan or D-phenylalanine amino acid residue;
Xaa2 is L-leucine or L-alanine amino acid residue;
Dab is L-2,4-diaminobutyric acid amino acid residue;
excluding CH₃(CH₂)₁₄C(O)-D-Phe-Dab-Dab-Leu-NH₂ lipopeptide,
wherein the lipopeptides may be in a free form or in form of any pharmaceutically or cosmetically acceptable salt.

2. Lipopeptides according to claim 1, **characterized in that** n=10-13.

3. Lipopeptides according to claim 2, **characterized in that** n=10 or n=12.

4. Lipopeptides according to claim 1, 2 or 3, **characterized in that** the Xaa1 amino acid residue is a D-phenylalanine residue.

5. Lipopeptides according to any one of claims 1 to 4, **characterized in that** the Xaa2 amino acid residue is an L-leucine residue.

6. A pharmaceutical composition comprising lipopeptides as defined in any one of claims 1 to 5.

7. The pharmaceutical composition according to claim 6, **characterized in that** it comprises 0.1 to 99% wt. of lipopeptides as defined in any one of claims 1 to 5.

8. The pharmaceutical composition according to claims 6 or 7, **characterized in that** it comprises pharmaceutically acceptable carriers and/or diluting agents and/or excipients and/or vehicles.

9. The pharmaceutical composition according to claim 8, **characterized in that** it comprises corn starch and/or gelatin and/or lactose and/or sucrose and/or microcrystalline cellulose and/or kaolin and/or mannitol and/or dicalcium phosphate and/or sodium chloride and/or alginic acid.

10. The pharmaceutical composition according to any one of claims 6 to 9, **characterized in that** it is in a form suitable for oral and/or intravenous and/or intramuscular and/or subcutaneous and/or parenteral administration.

11. The pharmaceutical composition according to claim 10, **characterized in that** it is in the form of tablets and/or capsules and/or elixir and/or suspension and/or syrup and/or gel and/or cream and/or ointment.

12. A cosmetic composition comprising lipopeptides as defined in any one of claims 1 to 5 in any cosmetically acceptable dosage form.

13. The cosmetic composition according to claim 12, **characterized in that** it comprises 0.1 to 99% wt. of lipopeptides as defined in any one of claims 1 to 5.

14. The cosmetic composition according to claims 12 or 13, **characterized in that** it comprises a cosmetic carrier.

15. The cosmetic composition according to claims 12 or 13 or 14, **characterized in that** it is in the form of gel or cream or suspension.

16. Lipopeptides as defined in any one of the claims 1 to 5 for use as a drug.

17. The lipopeptides as defined in claims 1 to 5 for treatment of bacterial infections, preferably caused by Gram-positive bacteria, preferably of the genus *Staphylococcus*.

## Patentansprüche

1. Lipopeptide der allgemeinen Formel 1:
**CH₃(CH₂)ₙC(O)-Xaa1-Dab-Dab-Xaa2-NH₂** (Formel 1)
worin
CH₃(CH₂)ₙC(O)- ein gesättigter Fettsäurerest ist, wobei n=10-14;
Xaa1 ein D-Tryptophan- oder D-Phenylalanin-Aminosäurerest ist;
Xaa2 ein L-Leucin- oder L-Alanin-Aminosäurerest ist;
Dab ein L-2,4-Diaminobuttersäure-Aminosäurerest ist;
ausgenommen CH₃(CH₂)₁₄C(O)-D-Phe-Dab-Dab-Leu-NH₂-Lipopeptid,
wobei die Lipopeptide in freier Form oder in Form eines pharmazeutisch oder kosmetisch verträglichen Salzes vorliegen können.

2. Lipopeptide nach Anspruch 1, **dadurch gekennzeichnet, dass** n = 10-13.

3. Lipopeptide nach Anspruch 2, **dadurch gekennzeichnet, dass** n=10 oder n=12.

4. Lipopeptide nach Anspruch 1, 2 oder 3, **dadurch gekennzeichnet, dass** der Xaa1-Aminosäurerest ein D-Phenylalaninrest ist.

5. Lipopeptide nach einem der Ansprüche 1 bis 4, **dadurch gekennzeichnet, dass** der Xaa2-Aminosäurerest ein L-Leucinrest ist.

6. Pharmazeutische Zusammensetzung, die Lipopeptide nach einem der Ansprüche 1 bis 5 umfasst.

7. Pharmazeutische Zusammensetzung nach Anspruch 6, **dadurch gekennzeichnet, dass** sie 0,1 bis 99 Gew. % Lipopeptiden enthält, wie sie in einem der Ansprüche 1 bis 5 definiert sind.

8. Pharmazeutische Zusammensetzung nach Anspruch 6 oder 7, **dadurch gekennzeichnet, dass** sie pharmazeutisch verträgliche Träger und/oder Verdünnungsmittel und/oder Hilfsstoffe und/oder Vehikel enthält.

9. Pharmazeutische Zusammensetzung nach Anspruch 8, **dadurch gekennzeichnet, dass** sie Maisstärke und/oder Gelatine und/oder Lactose und/oder Saccharose und/oder mikrokristalline Cellulose und/oder Kaolin und/oder Mannitol und/oder Dicalciumphosphat und/oder Natriumchlorid und/oder Alginsäure enthält.

10. Pharmazeutische Zusammensetzung nach einem der Ansprüche 6 bis 9, **dadurch gekennzeichnet, dass** sie in einer zur oralen und/oder intravenösen und/oder intramuskulären und/oder subkutanen und/oder parenteralen Verabreichung geeigneten Form vorliegt.

11. Pharmazeutische Zusammensetzung nach Anspruch 10, **dadurch gekennzeichnet, dass** sie in Form von Tabletten und/oder Kapseln und/oder Elixier und/oder Suspension und/oder Sirup und/oder Gel und/oder Creme und/oder Salbe vorliegt.

12. Kosmetische Zusammensetzung, die Lipopeptide nach einem der Ansprüche 1 bis 5 in jeder kosmetisch akzeptablen Dosierungsform umfasst.

13. Kosmetische Zusammensetzung nach Anspruch 12, **dadurch gekennzeichnet, dass** sie 0,1 bis 99 Gew. % Lipopeptiden enthält, wie sie in einem der Ansprüche 1 bis 5 definiert sind.

14. Kosmetische Zusammensetzung nach Anspruch 12 oder 13, **dadurch gekennzeichnet, dass** sie einen kosmetischen Träger enthält.

15. Kosmetische Zusammensetzung nach den Ansprüchen 12 oder 13 oder 14, **dadurch gekennzeichnet, dass** sie in Form eines Gels oder einer Creme oder einer Suspension vorliegt.

16. Lipopeptide nach einem der Ansprüche 1 bis 5 zur Verwendung als Arzneimittel.

17. Lipopeptide nach den Ansprüchen 1 bis 5 zur Behandlung bakterieller, vorzugsweise durch Gram-positive Bakterien verursachten, Infektionen, vorzugsweise der Gattung *Staphylococcus.*

## Revendications

1. Lipopeptides de Formule générale 1 :
**CH₃(CH₂)ₙC(O)-Xaa1-Dab-Dab-Xaa2-NH₂** (Formule 1)
où:
CH₃(CH₂)ₙC(O)- est un résidu d'acide gras saturé, dans lequel n = 10 à 14;
Xaa1 est un résidu d'acide aminé de D-tryptophane ou D-phénylalanine;
Xaa2 est un résidu d'acide aminé de L-leucine ou L-alanine;
Dab est un résidu d'acide aminé d'acide L-2,4-diaminobutyrique;
à l'exclusion du lipopeptide CH₃(CH₂)₁₄C(O)-D-Phe-Dab-Dab-Leu-NH₂,
dans lequel les lipopeptides peuvent être sous forme libre ou sous forme de tout sel pharmaceutiquement ou cosmétiquement acceptable.

2. Lipopeptides selon la revendication 1, **caractérisés en ce que** n = 10 à 13.

3. Lipopeptides selon la revendication 2, **caractérisés en ce que** n=10 ou n=12.

4. Lipopeptides selon la revendication 1, 2 ou 3, **caractérisés en ce que** le résidu d'acide aminé Xaa1 est un résidu de D-phénylalanine.

5. Lipopeptides selon l'une quelconque des revendications 1 à 4, **caractérisés en ce que** le résidu d'acide aminé Xaa2 est un résidu de L-leucine.

6. Composition pharmaceutique comprenant des lipopeptides tels que définis dans l'une quelconque des revendications 1 à 5.

7. Composition pharmaceutique selon la revendication 6, **caractérisée en ce qu'**elle comprend 0,1 à 99% en poids de lipopeptides tels que définis dans l'une quelconque des revendications 1 à 5.

8. Composition pharmaceutique selon les revendications 6 ou 7, **caractérisée en ce qu'**elle comprend des véhicules et/ou des agents diluants et/ou des excipients et/ou des véhicules pharmaceutiquement acceptables.

9. Composition pharmaceutique selon la revendication 8, **caractérisée en ce qu'**elle comprend de l'amidon de maïs et/ou de la gélatine et/ou du lactose et/ou du saccharose et/ou de la cellulose microcristalline et/ou du kaolin et/ou du mannitol et/ou du phosphate dicalcique et/ou du chlorure de sodium et/ou de l'acide alginique.

10. Composition pharmaceutique selon l'une quelconque des revendications 6 à 9, **caractérisée en ce qu'**elle se présente sous une forme adaptée à une administration orale et/ou intraveineuse et/ou intramusculaire et/ou sous-cutanée et/ou parentérale.

11. Composition pharmaceutique selon la revendication 10, **caractérisée en ce qu'**elle se présente sous forme de comprimés et/ou de gélules et/ou d'élixir et/ou de suspension et/ou de sirop et/ou de gel et/ou de crème et/ou de pommade.

12. Composition cosmétique comprenant des lipopeptides tels que définis dans l'une quelconque des revendications 1 à 5 sous toute forme posologique cosmétiquement acceptable.

13. Composition cosmétique selon la revendication 12, **caractérisée en ce qu'**elle comprend 0,1 à 99% en poids de lipopeptides tels que définis dans l'une quelconque des revendications 1 à 5.

14. Composition cosmétique selon les revendications 12 ou 13, **caractérisée en ce qu'**elle comprend un support cosmétique.

15. Composition cosmétique selon les revendications 12 ou 13 ou 14, **caractérisée en ce qu'**elle se présente sous forme de gel ou de crème ou de suspension.

16. Lipopeptides tels que définis dans l'une quelconque des revendications 1 à 5 pour une utilisation comme médicament.

17. Lipopeptides tels que définis dans les revendications 1 à 5 pour le traitement des infections bactériennes, de préférence provoquées par des bactéries Gram-positives, de préférence du genre *Staphylococcus*.
